# EUROPEAN PATENT APPLICATION

(11) **EP 4 166 136 A1**
(43) Date of publication of application: **19.04.2023**
(21) Application number: 21202711.4
(22) Date of filing: 14.10.2021
(51) Int. Cl.: A61K 31/336, A61P 11/00, A61P 11/06, A61P 35/04

(54) **INHALABLE PROTEASE INHIBITORS FOR USE IN THE PREVENTION AND/OR TREATMENT OF FIBROTIC AUTOIMMUNE OR INFLAMMATORY LUNG DISEASES**

(71) Applicant: Albert-Ludwigs-Universität Freiburg, 79098 Freiburg (DE)
(72) Inventor: FÄHNDRICH, Sebastian, 79463 Malsterdingen (DE); HUG, Martin J., 79114 Freiburg (DE); ZISSEL, Gernot, 79144 Freiburg (DE); PETERS, Christoph, 79353 Freiburg (DE)
(74) Representative: Krauss, Jan

(57) **Abstract**

The present invention relates to a compound according to formula (I) or a salt, solvate and/or a hydrate thereof, wherein said compound inhibits a cysteine protease, for use in the treatment and prevention of an inflammatory and/or other fibrotic lung disease, wherein said compound is administered by inhalation as a pharmaceutical composition, preferably comprising alcohol as a carrier.

## Description

The present invention relates to a compound according to formula (I) or a salt, solvate and/or a hydrate thereof, wherein said compound inhibits a cysteine protease, for use in the treatment and prevention of an inflammatory and/or other fibrotic lung disease, wherein said compound is administered by inhalation as a pharmaceutical composition, preferably comprising ethanol as a carrier.

### Background of the invention

In multiple pulmonary and inflammatory multi-system disorders, inflammation causes an initial injury of the lung tissue (acute lung injury) that leads to a progressive destruction ending with lung fibrosis, respiratory failure and death. No effective resounding drugs are currently available that stop the process of irreversible remodeling of the lungs.

Among various classes of proteases, there is a growing body of evidence that cysteine cathepsins participate in pulmonary homeostasis [1]. Although their exact functions remain to be clarified, cathepsins are potent ECM-degrading enzymes [2]. These enzymes belong to the family C1 (11 cysteine cathepsins for the human genome: cathepsins B, H, L, S, C, K, O, F, V, X, and W [3, 4] and have long been thought to be primarily involved in lysosomal end stage degradation of endocytosed proteins [5].

Cathepsins are located intracellularly in the endosomes / lysosomes of the cells and mainly act in an acidic pH environment. By processing and releasing proteins, cathepsins regulate the activation and release of inflammatory mediators and enzymes [6-10]. Cathepsins show effects that would counteract the common end of remodeling in all of the following diseases (autoimmune disorders and interstitial lung diseases that affect the lungs and induce a remodeling of the lung tissue up to pulmonary fibrosis as common trunk, systemic sclerosis, chronic hypersensitivity pneumonitis, idiopathic lung fibrosis, sarcoidosis, bronchiolitis obliterans syndrome (BOS) / graft versus host disease, chronic graft failure after lung transplantation, bronchial asthma, COPD and emphysema, check point inhibitor induced pneumonitis, cystic fibrosis lymphangioleiomyomatosis [11][11, 10] and in ARDS (including SARS-CoV-2 or influenza virus induced ARDS) [1, 5-8, 10-42].

The protease inhibitor aloxistatin (alternative names: E64D; EST; Estate; Loxistatin; Rexostatine) is a synthetic analogue of E 64, a natural product derived from fungi. E64D is the ethyl ester of E64C, and is hydrolyzed back to E64C as the more active form during the permeation through intestinal membrane (i.e. after oral intake). The chemical formula of aloxistatin is as follows:

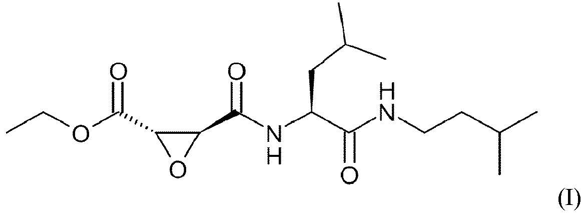

Chemically, E64D is an L-leucine derivative that is the amide obtained by formal condensation of the carboxy group of (2*S,*3*S*)-3-(ethoxycarbonyl)oxirane-2-carboxylic acid with the amino group of *N-*(3-methylbutyl)-L-leucinamide. It is thus an L-leucine derivative, a monocarboxylic acid amide, an epoxide and an ethyl ester. E64D is an inhibitor of cysteine proteases cathepsins B and L and is also thought to inhibit calpain.

E64D was initially developed for the treatment of muscular dystrophy, but was not successful in human clinical trials, though it has continued to be investigated for treatment of spinal cord injury, stroke, and Alzheimer's disease. E64D has also been described to have some antiviral effects [43].

WO2012112363 relates to deuterated compounds as cathepsin K inhibitors which are useful for treating diseases in which inhibition of bone resorption is indicated, such as osteoporosis [44].

[44] examined the putative role of cathepsin K (catK) in the pathology of lung fibrosis in mice and its relevance to the human disease activity.

There is a very urgent medical need for new drugs that prevent the fatal course of these various acute and chronic inflammatory diseases of any entity which, starting from an acute lung injury (ALI), lead to an irreversible remodeling of the lung tissue up to terminal fibrosis of the tissue resulting in the death of the patient. According to our own research results, E64D has the therapeutic potential of preventing from acute lung damage of any entity (regardless of whether it is viral, bacterial, autoimmune, noxious) in order to prevent irreversible damage to the lung tissue. With the efficient local deposition of E64D in the lungs, terminal remodeling processes of the lung tissue should avoid severe hypoxia with the necessity of a lung transplantation. Otherwise, patients often die from respiratory failure. By the inhibition of cysteine proteases in these different lung diseases, which begin like a common strain with an acute lung injury and lead to an irreversible remodeling of the lung tissue, the premature death of these patients should be avoided. Other objects and advantages will become apparent to the person of skill when studying the present description of the present invention.

In a first aspect of the present invention, this object is solved by a compound according to Formula (I) 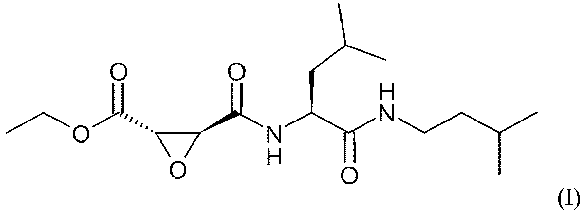 (aloxistatin, E64D; EST; Estate; Loxistatin; Rexostatine), a physiologically acceptable salt, a solvate, or a hydrate thereof for use in the prevention and/or treatment of an inflammatory and/or other fibrotic lung disease in a mammalian subject, such as a human, wherein said compound, the physiologically acceptable salt, the solvate, or the hydrate thereof is administered by inhalation as a pharmaceutical composition comprising a suitable solvent as a carrier.

In the context of the present invention, not only anti-inflammatory and antifibrotic properties of the active ingredient E64D with regard to remodeling of the lung tissue was shown, but also a direct antiviral effect in CoViD-19. In preclinical experiments, E64D was shown to inhibit viral entry into SARS-CoV-2 infected cell lines.

The compound according to Formula I (aloxistatin, E64D; EST; Estate; Loxistatin; Rexostatine) downregulates inflammation caused by the different disease entities and should prevent from ongoing inflammatory pathways ending in an irreversible fibrotic remodeling (our own preclinical experiments show antinflammatory and antifibrotic effects in vitro). In the present own preclinical experiments with rats, E64D shows low toxic effects when inhaled into the lungs. According to a preferred aspect of the invention, E64D is inhaled to prevent progression of the disease towards a more severe stage. E64D is inhaled because oral administration leads to an inactivation of aloxistatin (E64D). Orally administered, E64D is cleaved by ubiquitous esterases into its acid form, loxistatin (E64C). Since only the ester compound aloxistatin can pass the cellular membrane as a lipophilic pro-drug, and furthermore blocks intracellular cathepsins, a higher efficiency of the drug is ensured when bypassing the gastrointestinal tract which can be achieved by inhalation. In addition, the topical application using inhalation leads to higher local deposition than with oral gavage.

It was found in the context of the present invention that the substance E64D inhibits the migration of neutrophils from the vessels into the lung tissue. Neutrophils are inflammatory cells that release tissue-degrading enzymes in the lung tissue. The chemotactic migration of neutrophils from the vessels through the vessel walls into the lung tissue is mediated by pro-inflammatory mediators, such as interleukin-8, interleukin-17 and metalloproteinase-19. The release of interleukin-8 and metalloproteinase-19 takes place from the alveolar epithelial cells and from the vascular endothelial cells and is triggered by infection of these cells with viruses and / or interaction with bacteria and / or by environmental influences, such as fine dust deposition or physical stimuli such as ionizing radiation.

IL-17, on the other hand, is released from Th17 cells. A differentiation of the T-lymphocytes in the blood and in the lung tissue to Th17 cells occurs in autoimmune diseases (so-called collagenoses), granulomatous diseases (e.g. sarcoidosis), rejection reactions after lung transplantation, in bronchiolitis obliterans syndrome in the context of graft versus host disease, systemic sclerosis, hypersensitivity pneumonitis, Th17-associated neutrophilic bronchial asthma and in the adult respiratory distress syndrome (ARDS) (especially in CoViD-19 related ARDS). The Th17 cells secrete IL-17, which recruits neutrophil granulocytes from the blood vessels into the lung tissue leading to acute lung injury by the neutrophils' release of aggressive lung tissue destroying enzymes. These enzymes perpetuate inflammation and the recruitment of further inflammatory effector cells, including Th1- and Th17-lymphocytes that release additional pro-inflammatory mediators. These mediators contribute to so-called "acute phase reaction" leading to edema, increased vascular permeability with exudation. The mediators - especially IL-17 - are chemo-attractants for the recruitment of lung fibroblasts. The present inventors in their preclinical in vitro experiments could show that E64D inhibits the release of IL-8 and metalloproteinase from lung epithelial cells (A549). Moreover, evidence is provided that E64D inhibits the differentiation of T-lymphocytes into Th17-cells. E64D inhibited the release of proinflammatory IL-17 from Th17-cells. In preliminary experiments, it was shown that E64D is able to inhibit the IL-17 induced scarring of the tissue by inhibiting the IL-17 induced proliferation and differentiation of fibroblasts. Furthermore, inhibitory effects of E64D on the release of TGF-beta from fibroblasts, another mediator that causes progression of lung tissue fibrosis, is expected. The inventors were able to show in vitro that E64D inhibits the release of collagen from lung epithelial cells. Collagen plays a key role in terminal remodeling of the lung parenchyma through to total fibrosis.

To date, oral cathepsin inhibitors for other diseases have been the subject of clinical testing. These inhibitors only inhibited one of a large number of existing cathepsins. So far, no good efficacy was shown in clinical trials with either an orally applicable cathepsin B or a cathepsin C inhibitor. The systemic inhibition of several cathepsins, some of which also have redundant mechanisms of action, was previously considered too dangerous, since cathepsins also have important physiological properties, such as the antigen presentation on the MHC II complex on macrophages. In addition to the dreaded systemic effect of broad-spectrum cathepsin inhibitors, the pharmaceutical challenge is that there is no stable orally available broad-spectrum cathepsin inhibitor. The present invention overcomes these problems.

One of the advantages of the inhalable administration of E64D is that the local administration with direct inactivation of the substance in the tissue target cells prevents from the dangerous systemic effect of the broad cathepsin inhibitor E64D (inhibition of cathepsins B, H, K, L and S). In addition, due to the local deposition in the lungs, the inactivation of the substance by esterases in the intestine and in the blood is bypassed.

It is expected that a much more efficient anti-inflammatory, anti-viral and anti-fibrotic effect can be achieved through the simultaneous inhibition of several synergistically acting cathepsins. The inhibition of different cathepsins simultaneously enables further the treatment of several disease entities, which, via a different pathomechanism, lead to acute lung injury with subsequent chronic inflammation of the lung tissue including terminal fibrosis. The present invention provides these advantages.

It was not possible to treat the various disease entities and conditions that include/lead to acute and chronic inflammation with fibrosis of the lung tissue using previously clinically tested and orally / systemically administered substances that only inhibit individual cathepsins, and the present invention now provides the inhalable, broad-acting cathepsin inhibitor E64D for a wide spectrum of diseases that lead to terminal remodeling of the lungs.

In the context of the present invention, the term "aloxistatin" or "E64D" shall include the compound according to formula I in its physiologically acceptable salt forms, such as the calcium, potassium, magnesium, choline or sodium salt. The term shall also include physiologically acceptable solvates, and hydrates. As a prodrug, it shall also include E64C, which seems to be the more active form following the permeation through intestinal membrane (i.e., after oral uptake). The chemical formula of E64C is as follows:

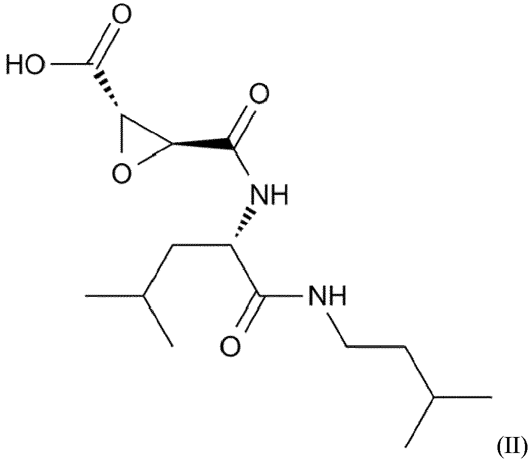

According to the present invention, "an inflammatory and/or other fibrotic lung disease" is a disease or condition of the lung that includes an initial injury of the lung tissue leading to a progressive destruction of the lung tissue and involving an irreversible process of remodeling that leads to a fibrosis of the lung. Preferred is therefore a compound for use according to the present invention, wherein said inflammatory and/or other fibrotic lung disease is an interstitial and/or neoplastic autoimmune lung disease, and/or said prevention and/or treatment is against exacerbations or severe inflammation in said disorder, and/or to inhibit the IL-17 induced scarring of the lung tissue, preferably to avoid irreversible airway remodeling up to terminal fibrosis of the lung.

Further preferred is a compound for use according to the present invention, wherein the use excludes treatment or prevention of a viral infection by a virus selected from the viral group of betacoronavirus, such as HCoV-OC43, SARS-CoV-1, HCoV-HKU1, MERS-CoV or SARS-CoV-2 and/or the G614 variant thereof.

According to the present invention, a mammalian subject or patient can be preferably selected from a mouse, rat, cat, dog, rabbit, goat, sheep, horse, camel, lama, cow, monkey, a farm animal, a sport animal, and a pet, and a human.

According to the present invention, the compound preferably is for use in the prevention and/or treatment of an inflammatory and/or other fibrotic lung disease in a mammalian subject selected from the group consisting of acute or chronic inflammation of the lung tissue that can be associated with fibrosis of the lung tissue, such as, for example, idiopathic pulmonal fibrosis (IPF), non-specific interstitial pneumonia (NSIP); acute interstitial pneumonia (AIP), lymphoid interstitial pneumonia (LIP), respiratory bronchiolitis with interstitial lung disease (RB-ILD), histocytosis, fibrotic autoimmune disorders, systemic sclerosis, rheumatoid arthritis with fibrotic lung involvement, asthma bronchiale, in particular Th1- and/or Th2- and/or Th17-related asthma bronchiale, Sjogren's syndrome, antinuclear antibody (ANA) and anti-neutrophil cytoplasmic antibody (ANCA) associated vasculitis, lupus erythematosus with fibrotic lung involvement, hypersensitivity pneumonitis, Granulomatosis with polyangiitis, eosinophilic granulomatosis and polyangiitis, sarcoidosis, beryllium disease, cystic fibrosis, check point inhibitor induced pneumonitis, radiation induced pneumonitis, graft versus host disease, bronchiolitis obliterans syndrome, chronic lung allograft dysfunction, IgG4-associated diseases of the lungs, lymphangioleiomyomatosis, amyloidosis of the lungs, lung metastases derived from lung cancer, breast cancer, colon cancer or renal cell cancer, chronic obstructive pulmonary disorder (COPD), emphysema, and acute respiratory distress syndrome (ARDS) in patients with severe viral and bacterial pneumonia.

By "treatment" or "treating" is meant any treatment of a disease or disorder, in a mammal, including: preventing or protecting against the disease or disorder, that is, causing, the clinical symptoms of the disease not to develop; inhibiting the disease, that is, arresting or suppressing the development of clinical symptoms; and/or relieving the disease, that is, causing the regression of clinical symptoms. By "amelioration" is meant the prevention, reduction or palliation of a state, or improvement of the state of a subject; the amelioration of a stress is the counteracting of the negative aspects of a stress. Amelioration includes but does not require complete recovery or complete prevention of a stress.

In another important aspect according to the present invention regarding the compound for use, said prevention and/or treatment is in combination with another suitable anti-inflammatory and/or antiinfective therapy, for example selected from at least one remdesivir, interferon alpha 2a or 2b, inclusive any pegylated versions, chloroquine or hydroxychloroquine, serine protease inhibitors, cysteine protease inhibitors, and/or type II transmembrane protease (TMPRSS2) inhibitors, in particular camostat ((4-{2-[2-(Dimethylamino)-2-oxoethoxy]-2-oxoethyl}phenyl)(4-carbamimidamidobenzoate), inhalable corticosteroids, vasointestinal peptide (VIP) and furin inhibitors.

The compound for use is provided and/or is administered as a suitable pharmaceutical composition for inhalation, such as a dry powder inhaler or other inhalation forms. Such formulations may comprise excipients and other ingredients in suitable amounts. The combination compound is provided as a suitable pharmaceutical composition, such as a tablet, capsule, injection, granule, powder, sachet, reconstitutable powder, dry powder inhaler, inhalation, and/or chewable.

Thus, the compounds for use of the invention can be used alone or in combination with other active compounds - for example with the aforementioned compounds, whereby in the latter case a favorable additive, amplifying or preferably synergistically effect is noticed. Suitable amounts to be administered to humans range from 1 to 500 mg, in particular 5 mg to 100 mg, such as between 1 and 10 mg/kg/day E64D dose. An effective concentration to be reached at the cellular level can be set at between 50 to 200 µM, preferably at about 100 µM. For topical application, such as to the airways by inhalation, the dosage can be conveniently reduced to between 0.1 to 10 mg/dose, preferably 0.2 to 5 mg per dose, which equals about 3 to about 80 µg per kilogram for a 70 kg subject.

In the context of the present invention, an efficient inhalation furthermore requires the presence of a suitable solvent. The choice is not trivial, as for an effective inhalation it is required that the drug does not precipitate and stays in solution. Due to the lipophilic nature of the compound only organic solvents can be used for a liquid formulation. Preferably, alcohol is used as a suitable solvent, the inventors found that ethanol, which itself has a disinfecting and antiviral effect, proved to be a suitable with a solubility of E64D of up to 68 mg/mL (198.58 mM). In addition, ethanol can be obtained in purified/GMP grade quality and is approved as additive for use in humans. The person of skill can identify respective concentrations that are suitable for the purposes of the present application.

It is to be understood that the present compound and/or a pharmaceutical composition comprising the present compound is for use to be administered to a human patient. The term "administering" means administration of a sole therapeutic agent or in combination with another therapeutic agent. It is thus envisaged that the pharmaceutical compositions of the present invention are employed in co-therapy approaches, i.e., in co-administration with other medicaments or drugs and/or any other therapeutic agent which may be beneficial in the context of the methods of the present invention. Nevertheless, the other medicaments or drugs and/or any other therapeutic agent can be administered separately from the compound for use, if required, if they act in combination (i.e., directly and/or indirectly, preferably synergistically) with the present compound for use.

In the context of the present invention, an efficient inhalation furthermore requires the choice of a suitable volume to be nebulized and inhaled, and thus the resulting drug concentration, on the one hand providing a sufficient dose in all sections of the respiratory tract (nasopharynx, upper, middle respiratory airways, and alveolar surface). On the other hand, the medical limits for the inhalation of ethanol must be considered, while precipitation must be avoided. The person of skill can identify respective volumes and concentrations that are suitable for the purposes of the present invention. Also, patient compliance is essential, in view of difficulties with breathing in case of progressed diseases.

Another important aspect of the present invention is the choice of the technique for inhalation. A preferred technology should avoid a loss of drug during inhalation and at the same time ensure a sufficient dose in all targeted sections of the respiratory system. Preferred is inhalation using a modern nebulizer, such as, for example the I-neb^{®} dose+ from Nebu-tec (NEBU-TEC, Elsenfeld, Germany), since this inhalation device allows breath-triggered inhalation without loss of the active ingredient to the environment. The inhalation device should ensure that the dose is deposited in the lungs. Data on the active ingredient dose, solution and particle size, and lung deposition of the inhalable aloxistatin can be obtained and controlled using common impactor measurements.

Pharmaceutical compositions as used may optionally comprise a pharmaceutically acceptable carrier. Pharmaceutically acceptable carriers or excipients include diluents (fillers, bulking agents, e.g. lactose, microcrystalline cellulose), disintegrants (e.g. sodium starch glycolate, croscarmellose sodium), binders (e.g. PVP, HPMC), lubricants (e.g. magnesium stearate), glidants (e.g. colloidal Si0₂), solvents/cosolvents (e.g. aqueous vehicle, Propylene glycol, glycerol, alcohol), buffering agents (e.g. citrate, gluconates, lactates), preservatives (e.g. Na benzoate, parabens (Me, Pr and Bu), BKC), anti -oxidants (e.g. BHT, BHA, Ascorbic acid), wetting agents (e.g. polysorbates, sorbitan esters), thickening agents (e.g. methylcellulose or hydroxyethylcellulose), sweetening agents (e.g. sorbitol, saccharin, aspartame, acesulfame), flavoring agents (e.g. peppermint, lemon oils, butterscotch, etc.), humectants (e.g. propylene, glycol, glycerol, sorbitol). Other suitable pharmaceutically acceptable excipients are inter alia described in Remington's Pharmaceutical Sciences, 15th Ed., Mack Publishing Co., New Jersey (1991) and Bauer et al., Pharmazeutische Technologie, 5th Ed., Govi-Verlag Frankfurt (1997). The person skilled in the art knows suitable formulations for E64D and will readily be able to choose suitable pharmaceutically acceptable carriers or excipients, depending, e.g., on the formulation and administration route of the pharmaceutical composition.

Yet another important aspect of the present invention is the choice of a suitable particle size that is aerodynamically suitable to reach all the targeted sections of the respiratory system (see above), while at the same time ensuring satisfying dosage deposition. The person of skill can identify respective sizes and particles that are suitable for the purposes of the present invention (see, for particles, for example, El-Sherbiny, Ibrahim M et al. "Inhaled nano- and microparticles for drug delivery." Global cardiology science & practice vol. 2015 2. 31 Mar. 2015, doi:10.5339/gcsp.2015.2). Suitable average particle diameter sizes are between about 2 and about 7 µm, preferably between about 3 and about 5 µm.

In the context of the present invention, the term "about" shall mean +/- 10% of a given value, unless noted otherwise.

In addition to the aforementioned compounds for use of the invention, the pharmaceutical composition can contain further customary, usually inert carrier materials or excipients. Thus, the pharmaceutical preparations can also contain additives, such as, for example, fillers, extenders, disintegrants, binders, glidants, wetting agents, stabilizers, emulsifiers, preservatives, sweetening agents, colorants, flavorings or aromatizers, buffer substances, and furthermore solvents or solubilizers or agents for achieving a depot effect, as well as salts for changing the osmotic pressure, coating agents or antioxidants. They can also contain the aforementioned salts of two or more compounds for use of the invention and also other therapeutically active substances as described above.

Preferred is the compound for use according to the present invention, wherein said compound is administered to said subject in an effective dosage. This dosage can vary within wide limits and is to be suited to the individual conditions in each individual case. For the aforementioned uses, the appropriate dosage will vary depending on the mode of administration (here, inhalation), the particular condition to be treated and the effect desired. In general, however, satisfactory results are achieved at dosage rates are as above, e.g., of about 1 to 100 mg/kg animal body weight particularly 1 to 50 mg/kg. Suitable dosage rates for larger mammals such as humans, are in the order of about 5 mg to 3 g/day, conveniently administered once or in divided doses, e.g., 2 to 4 times a day, preferably 3 time as day, or in sustained release form. In general, a daily dose of approximately 10 mg to 100 mg, particularly 10 to 50 mg, per human individual is appropriate in the case of the oral administration. An effective concentration to be reached at the cellular level can be set at between 50 to 200 µM, preferably at around 100 µM. Particularly preferred is topical application, such as to the airways by inhalation. In these cases, the dosage can be conveniently reduced to between 0.1 to 10 mg/dose, preferably 0.2 to 5 mg per dose, which equals about 3 to about 80 µg per kilogram for a 70 kg subject.

Another aspect thereof, the present invention provides methods for preventing and/or treating of an inflammatory and/or other fibrotic lung disease in a mammalian subject, such as a human, comprising administering to said mammal an effective amount of a compound according to Formula (I), 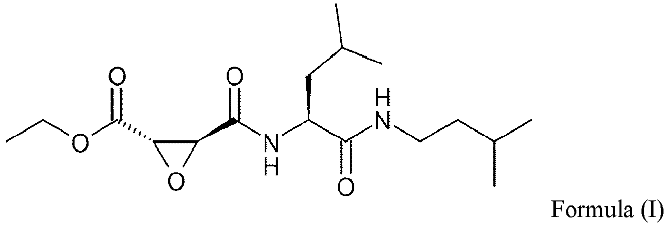 a physiologically acceptable salt, a solvate, or a hydrate thereof, wherein said compound is administered by inhalation as a pharmaceutical composition, preferably comprising alcohol as a carrier, such as ethanol.

Preferred is the method according to the present invention, wherein said inflammatory and/or other fibrotic lung disease is an interstitial and/or neoplastic autoimmune lung disease, and/or said prevention and/or treatment is against exacerbations or severe inflammation in said disorder, and/or to inhibit the IL-17 induced scarring of the lung tissue, and preferably to avoid irreversible airway remodeling up to terminal fibrosis of the lung.

Further preferred is the method according to the present invention, wherein said disease or condition is selected from the group consisting of lung fibrosis, such as, for example, IPF, NSIP; AIP, LIP, RB-ILD, histiocytosis, fibrotic autoimmune disorders, systemic sclerosis, rheumatoid arthritis with fibrotic lung involvement, asthma bronchiale, in particular Th1- and/or Th2- and/or Th17-related asthma bronchiale, Sjogren's syndrome, ANA and ANCA associated vasculitis, lupus erythematosus with fibrotic lung involvement, hypersensitivity pneumonitis, Granulomatosis with polyangiitis, eosinophilic granulomatosis and polyangiitis, sarcoidosis, beryllium disease, cystic fibrosis, check point inhibitor induced pneumonitis, radiation induced pneumonitis, graft versus host disease, bronchiolitis obliterans syndrome, chronic lung allograft dysfunction, IgG4-associated diseases of the lungs, lymphangioleiomyomatosis, amyloidosis of the lungs, lung metastases derived from lung cancer, breast cancer, colon cancer or renal cell cancer, COPD, emphysema, and ARDS in patients with severe viral and bacterial pneumonia.

In another preferred of the method according to the present invention, said prevention and/or treatment is in combination with another therapy, for example selected from at least one of interferon alpha 2a or 2b, inclusive any pegylated versions, serine protease inhibitors, cysteine protease inhibitors, and/or type II transmembrane protease (TMPRSS2) inhibitors, in particular camostat, inhalable corticosteroids, vasointestinal peptide (VIP) and furin inhibitors.

In the method, the compound for use can be provided and/or is administered as a suitable pharmaceutical composition as discussed above. The compounds can be administered alone or in combination with other active compounds - for example with medicaments already known for the treatment of the aforementioned diseases, whereby in the latter case a favorable additive, amplifying or preferably synergistically effect is noticed. Suitable amounts and dosages to be administered to mammals, in particular humans, are as above.

In a preferred embodiment of the method according to the present invention, the compound is administered to said subject in an effective dosage, for example of between 0.1 to 10 mg/dose, preferably 0.2 to 5 mg per dose, which equals about 3 to about 80 µg per kilogram for a 70 kg subject per inhalation.

In a preferred embodiment of the method according to the present invention, the method further comprises a monitoring step comprising an analysis selected from serum chemistries including electrolytes, renal function tests, such as creatinine, CrCL, and BUN, liver function tests, such as ALT, AST, total bilirubin, and alkaline phosphatase, hematology, such as complete blood count and prothrombin time, and urinalysis in a sample obtained from said mammalian subject, and comparing said analysis to the analysis of an earlier sample from said mammal and/or a control sample.

The present invention will now be described further in the following examples with reference to the accompanying Figures, nevertheless, without being limited thereto. For the purposes of the present invention, all references as cited herein are incorporated by reference in their entireties.

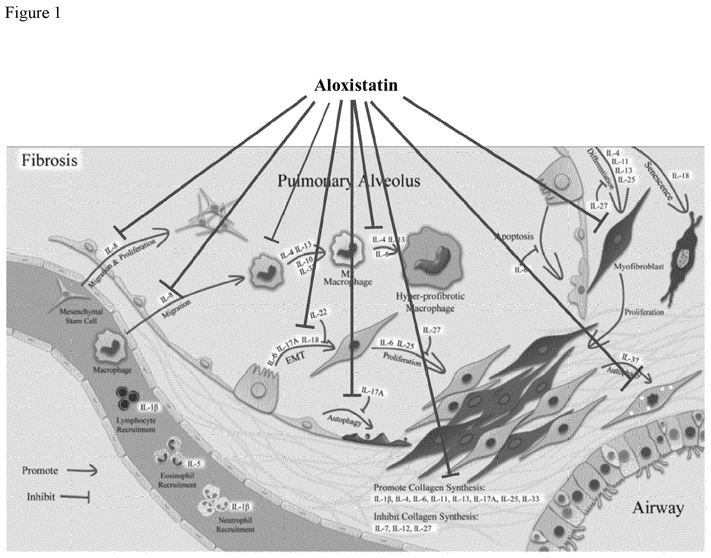

Figure 1 shows a schematic overview of the prospective effects of the cathepsin inhibitor aloxistatin (E64D) on fibrosis of the lung parenchyma as the final route of various inflammatory processes in the lungs. Figure modified from a template by She et al. (She YX et al.) Role of interleukins in the pathogenesis of pulmonary fibrosis. Cell death discovery. 2021;7(1):52). Interleukins influence the morphology and functions of various cells in pulmonary fibrosis. (1) Mesenchymal stem cells: IL-8 facilitates the migration and proliferation of mesenchymal stem cells. (2) Macrophages: IL-8 also induces migration of macrophages. IL-4, IL-13, IL-10, and IL-33 promote macrophages to transform into M2 phenotype, which is further promoted by IL-4, IL-6, and IL-13 to transform into a hyper-profibrotic phenotype. (3) Other immune cells: IL-1β induces recruitment of lymphocytes and neutrophils, while IL-5 induces recruitment of eosinophils. (4) Alveolar epithelial cells: IL-17A inhibits autophagy of alveolar epithelial cells, and IL-6 suppresses apoptosis of alveolar type II cells (AT II). IL-6, IL-17A, and IL-18 promote EMT of AT II, whereas IL-22 inhibits this process. (5) Fibroblasts: IL-6 and IL-25 promote the proliferation of fibroblasts. IL-4, IL-11, IL-27 inhibit collagen synthesis 13, and IL-25 induce differentiation of fibroblasts, whereas IL-27 suppresses both events. IL-18 contributes to the senescence of fibroblasts, and IL-37 facilitates autophagy of fibroblasts. IL-1β, IL-4, IL-6, IL-11, IL-13, IL-17A, IL-25, and IL-33 promote collagen synthesis, while IL-7, IL-12, and IL-27 inhibit collagen synthesis [45].

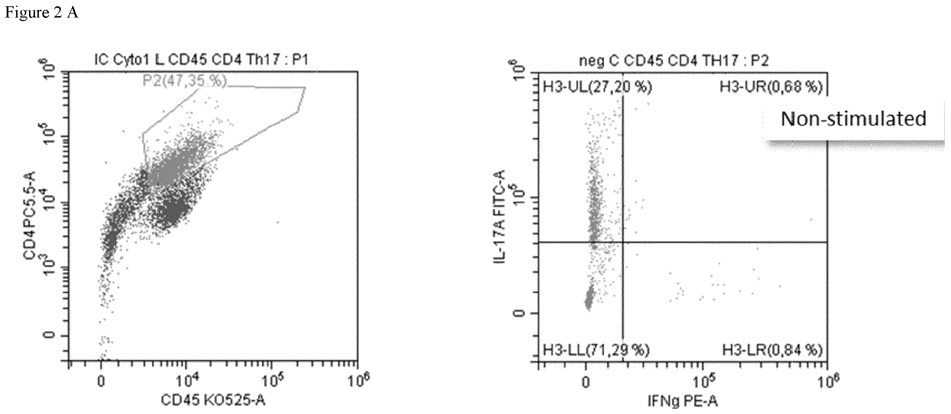

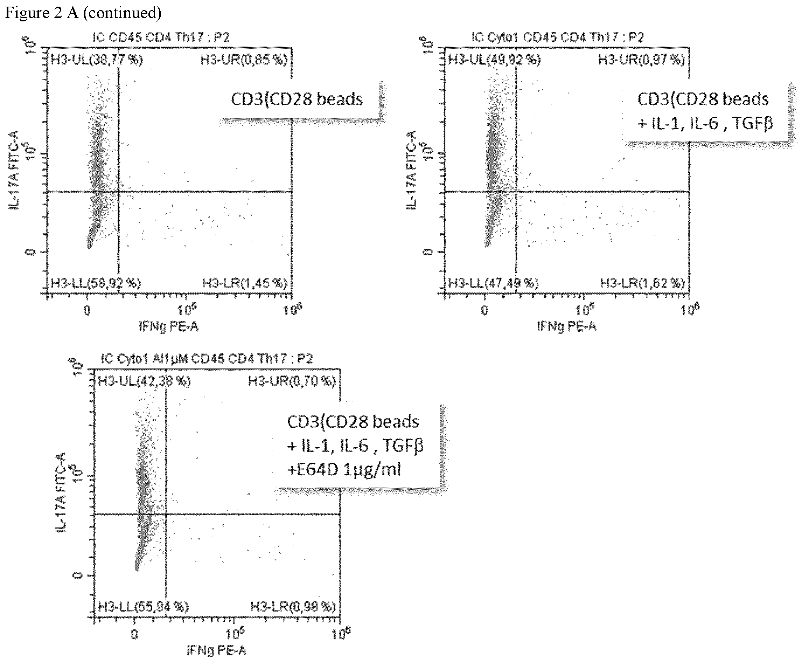

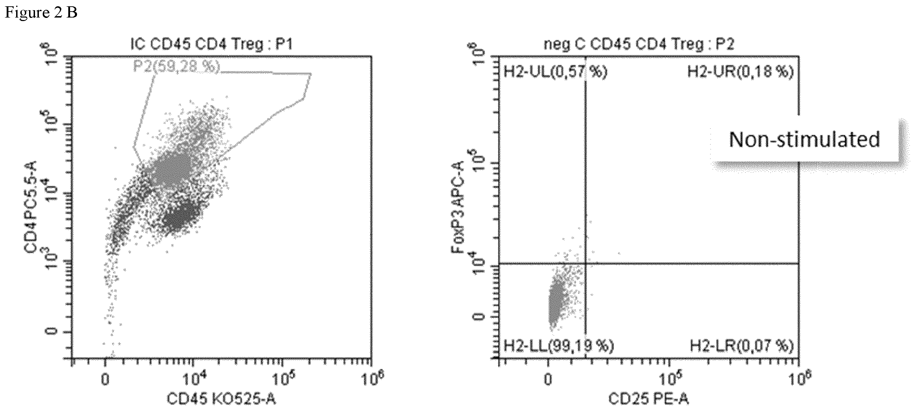

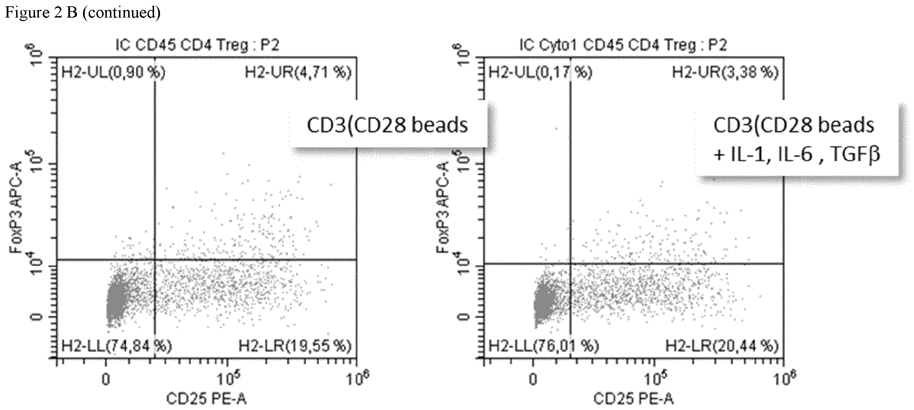

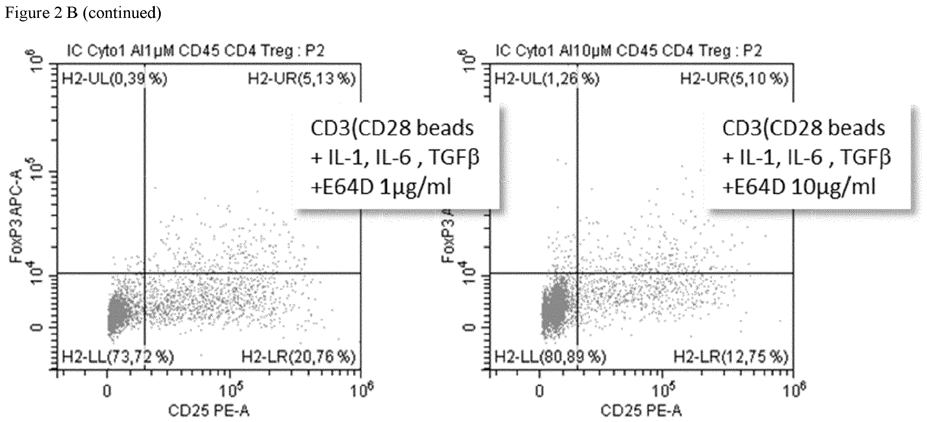

Figure 2 A and B shows the results of a measurement of Th17-lymphocytes by flow cytometry: Stimulation with IC-beads up-regulates Th17 (IL-17A) and slightly Th1 (IFN-gamma) cells. Addition of the cytokine mix increases the percentage of Th17 cells. In both cases, E64D reduces the percentage of Th17 cells. (A: whole blood lymphocytes, B: T-lymphocytes from bronchoalveolar lavage.

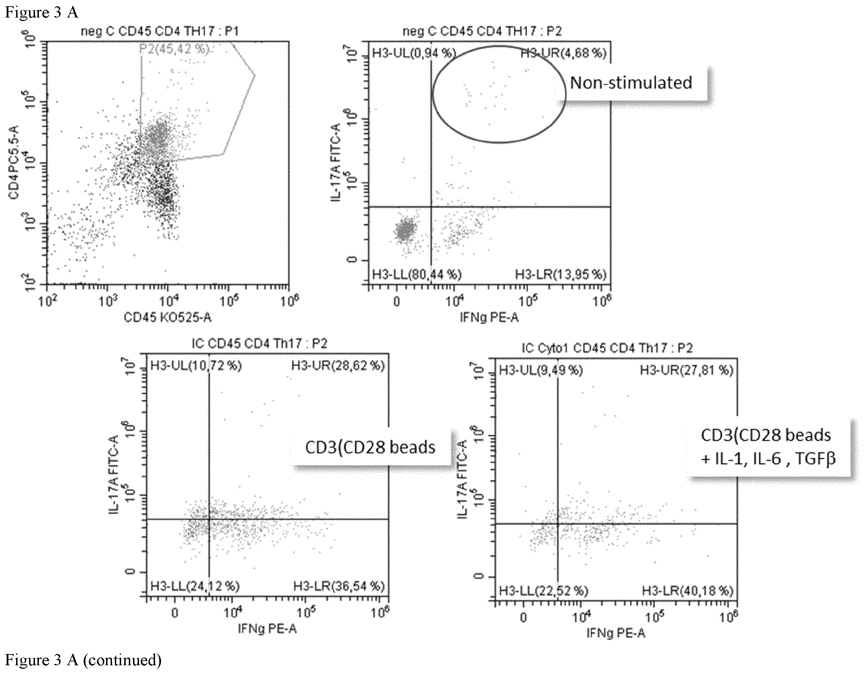

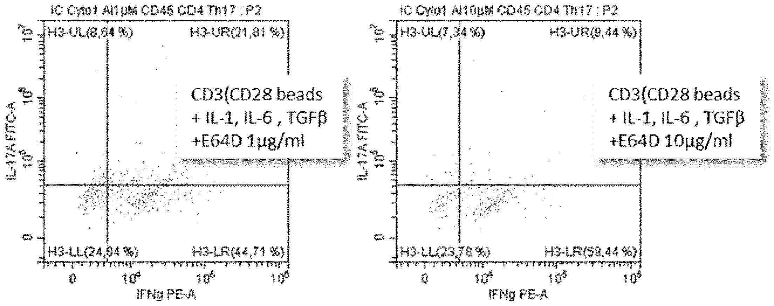

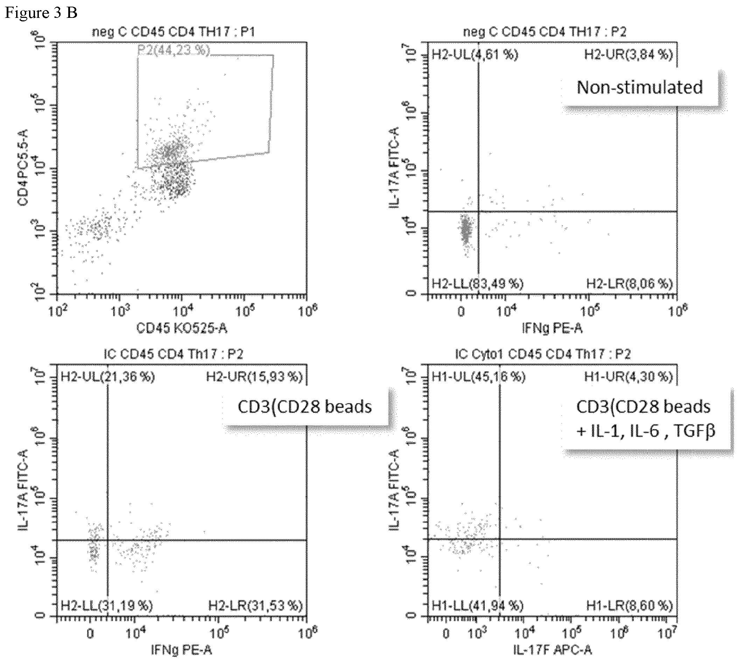

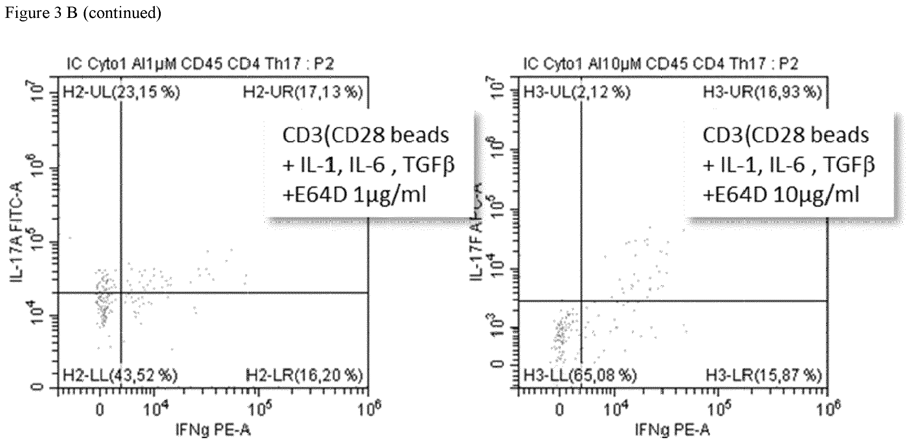

Figure 3 A and B shows the results of flow cytometry measurements showing a downregulation of Th17 lymphocytes and an upregulation of Treg lymphocytes after co-incubation with E64D. E64D dose-dependently down-regulates Th17 and Th17.1 cells. E64D dose-dependently up-regulates Th1 cells.

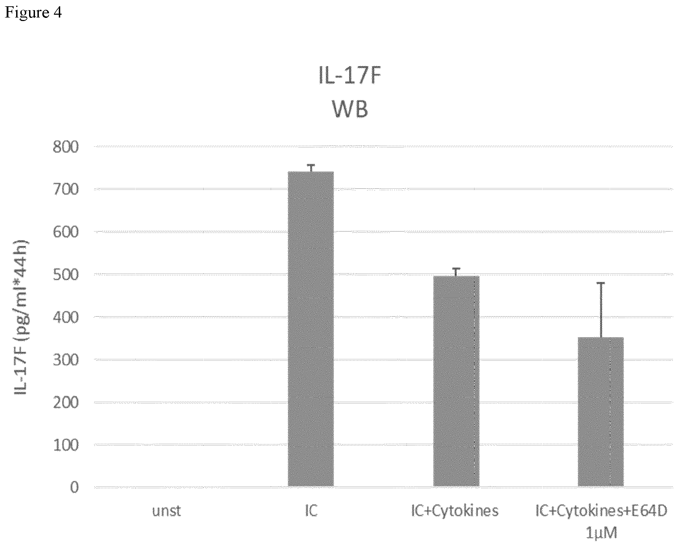

Figure 4 shows the inhibition of IL-17 release from human mononuclear cells in whole blood stimulated with cytokines.

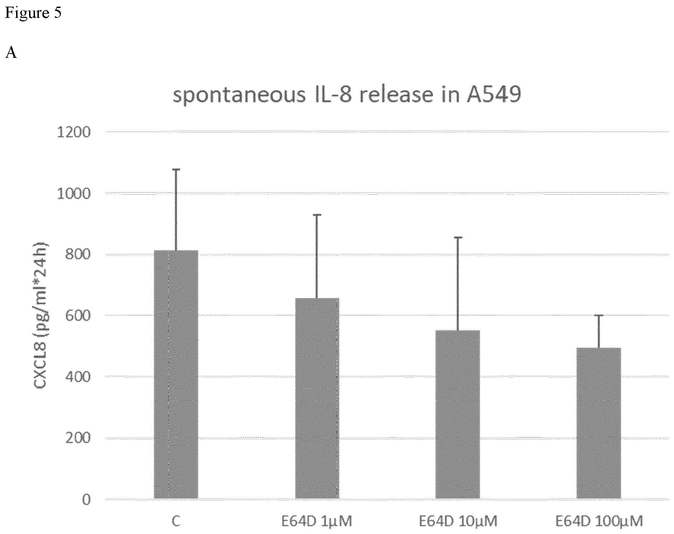

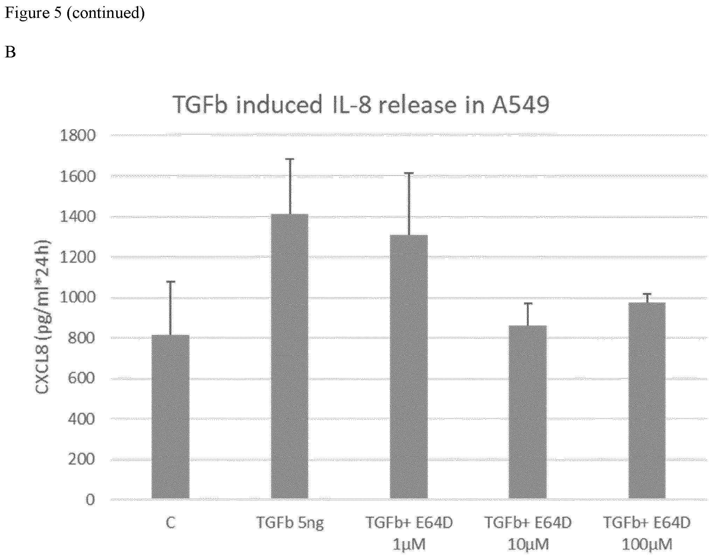

Figure 5 shows the results of experiments with A549 cells that were harvested, counted; and a defined number of cells was sown. The cells were given time to adhere overnight, and then a change of medium was carried out and after no stimulation or stimulation with TGFbeta the cells were cultivated in each case with and without E64D for 24 h. The supernatants were then removed and the IL-8 as release measured. E64D lowers both the spontaneous (A) and TGFbeta-induced IL-8 (B) release. E64D inhibited the spontaneous release of IL-8in a dose-dependent manner, while a concentration of 10 µg/ml was sufficient to block the induction almost completely.

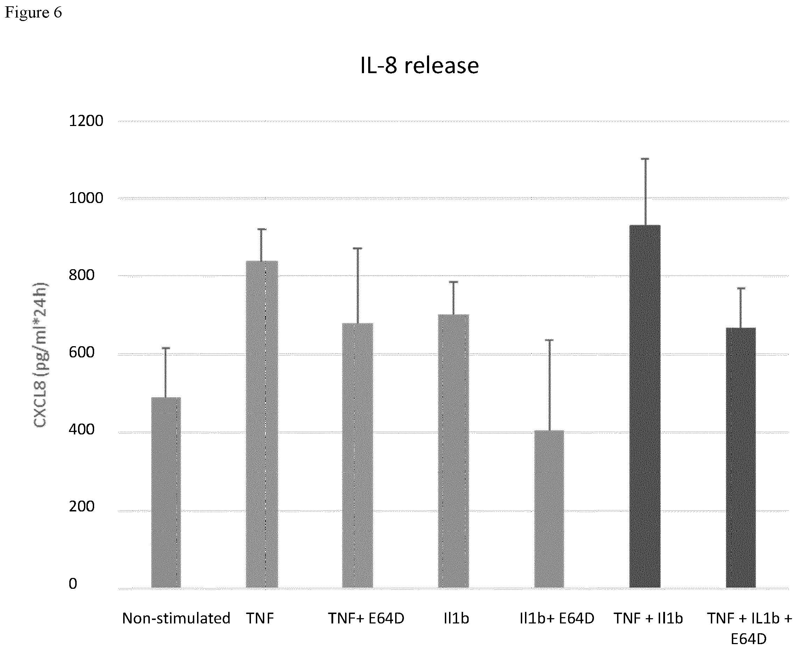

Figure 6 shows experiments with THP1 cells (macrophage-like cell line) that were stimulated with TNF, IL-1 or both and cultured with and without E64D for 24 hours. This stimulation corresponds to a pro-inflammatory response. The supernatants were then removed, and the IL-8 release was measured. E64D clearly lowers the IL-8 release in the IL-1 or TNF / IL-1 stimulated cells.

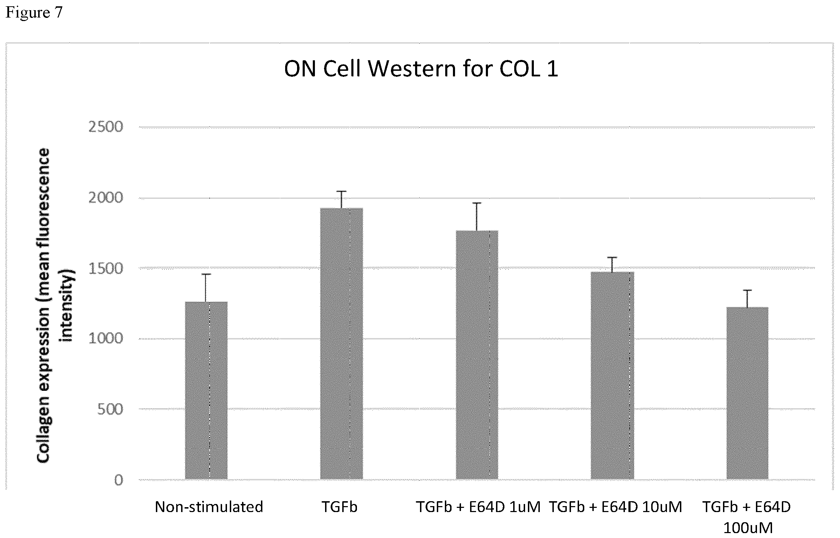

Figure 7 shows the results with A549 cells that were stimulated with TGFbeta and each cultured with and without E64D in various concentrations for 72 hours. Then, the cells were fixed with paraformaldehyde, incubated with rabbit anti-collagen I antibodies, and the bound antibodies were labeled with goat anti-rabbit were detected with IRDye 800CW on a Licor Odyssey near infrared scanner. E64D lowers the TGFbeta-induced collagen production of A549 in a dose-dependent manner. In the highest dose, the release almost goes back to the initial value. A second experiment showed similar results.

### Examples

While the examples have been performed using the preferred compound E64D, the person of skill will be readily able to adjust the experimental conditions to other cathepsin inhibitors that also fall into the scope of the present invention. See, for example, Banerjee et al. Development of potent and selective Cathepsin C inhibitors free of aortic binding liability by application of a conformational restriction strategy, Bioorganic & Medicinal Chemistry Letters, Volume 47, 2021, https://doi.org/10.1016/j.bmcl.2021.128202.

### Cytotoxicity of E64D

E64D was found to be non-toxic in a cellular model (Vero e6-cells) in concentrations up to 100 µM.

### Inhibition of Th17 driven inflammation by E64D

The Th17 driven inflammation is a hallmark in autoimmune disorders (e.g. systemic sclerosis, but also other autoimmune disorders and interstitial lung diseases that can affect the lungs and induce a remodeling of the lung tissue up to pulmonary fibrosis), chronic hypersensitivity pneumonitis, idiopathic lung fibrosis, sarcoidosis, bronchiolitis obliterans syndrome (BOS)/graft versus host disease, chronic graft failure after lung transplantation, bronchial asthma, cystic fibrosis, and in ARDS (including SARS-CoV-2 or influenza virus induced ARDS) [36, 38, 46, 47].

A Th17 driven inflammation cannot be suppressed by corticosteroids, and therefore, it is a challenge to successfully treat such an inflammatory phenotype [36]. The broad spectrum cathepsin inhibitor E64D is a candidate for a promising successful therapy since it suppresses Th17 differentiation in a dose-dependent manner in rats [37]. E64D inhibits cathepsins L, B, K, and S that actively regulate the differentiation of CD4 cells into Th17 cells through cytokines and transcription factors. The present experiments showed that aloxistatin inhibits Th17-differentiation and thus the release of the cytokine IL-17. See Figure 2 for results with human mononuclear (CD14)-Cells, where E64D reduces the percentage of Th17 cells.

Targeting cathepsins L, B, K, and S is also an approach for treating Th17 cell-driven autoimmune disorders and ARDS caused by virus pneumonia. Elevated TH17 (as well as TH1) responses or enhanced IL-17-related pathways are also observed in MERS-CoV and SARS-CoV infected patients. Patients with CoViD-19 associated ARDS have even higher levels of Th17 cytokines, both promote TH17 responses and vascular permeability and leakage which is important in the exudative phase of ARDS caused by SARS-CoV-2, where leukocytes extravasate into the interstitium of the lungs. The differentiation into TH17 cells themselves leads to a production of additional proinflammatory cytokines that perpetuate recruitment and activation of additional inflammatory cells in the lung tissue. Taken together, the TH17 type response contributes to the cytokine storm in pulmonary viral infection including SARS-CoV-2, which results in tissue damage and likely promotes pulmonary edema; E64D targets the TH17 pathway and patients with TH17 dominant immune profiles will benefit from a treatment with E64D.

E64D does not only downregulate Th17 lymphocytes, but also induces a shift to regulatory Treg cells (own preclinical experiments) (Figure 3), which are of great importance for the control of immune reactions by cytotoxic T cells. The inventors' findings in the field of interstitial lung diseases indicate that the increase in Treg lymphocytes is associated with a better course of the disease. The results also show that the release of the cytokine IL-17, which is associated with lung failure in the context of ARDS or in inflammatory interstitial lung diseases, can be inhibited by E64D (Figure 4).

All diseases to be treated in the context of the present invention have in common that initially a chemotactic migration of neutrophils from the blood into the tissue occurs. This migration is mediated by cytokines IL-17, IL-8, and metalloproteinase MMP-9. In the present experiments, the inventors were able to show that both IL-17 secretion and IL-8 secretion and the release of the metalloproteinase MMP-9 from mononuclear cells and alveolar epithelial cells (A549) are inhibited (Figures 4 and 5, as well as 6 for THP1 cells). Since the chemotactic migration of the neutrophil granulocytes in all disease entities initially triggers an ALI (acute lung injury) through the release of neutrophil elastase and DNA fragments, which in turn heats up the activation of further immune effector cells, the medical use of E64D does clearly provide a benefit in the diseases mentioned herein.

### Inhibition of lung fibrosis using E64D

In the lung, a fibrosis is accompanied by abnormal proteolytic activities. Pharmacological inhibition of cathepsin B slows down fibrosis due to diminishing smooth muscle actin expression and delaying fibroblast differentiation, associated with an impaired secretion of TGF-beta. Cathepsin inhibitors seem to selectively block NLRP3 and IL-1β secretion and contribute to abolishing injury due to progressive remodeling of the lung tissue (not shown in Figure 1). NLRP3 (NOD-, LRR- and pyrin domain-containing protein 3) is an intracellular sensor that detects a broad range of microbial motifs, endogenous danger signals and environmental irritants, resulting in the formation and activation of the NLRP3 inflammasome. Assembly of the NLRP3 inflammasome leads to the release of the pro-inflammatory cytokines IL-1β and IL-18, as well as pyroptotic cell death, and [48][47][47][47][47][47][46][46][45] (Swanson et al. 2019) E64D might act antinflammatory by inhibiting pro-IL-1β synthesis and NLRP3 activation.

Another anti-fibrotic property of E64D is the observed inhibition of calpain. Via release of TGF-β1, calpain activation augments collagen-I synthesis in fibroblasts and thus contributes to pulmonary fibrosis. The inhibition of calpain activation and TGF-β1 by E64D thus is a potential target for the treatment of fibrosis.

The experiments of the inventors showed that E64D inhibits the collagen release from alveolar epithelial cells (A549). Collagen release was measured because of its contribution to the development of lung fibrosis regardless of the cause of inflammation.

### Inhibition of ENaC and prevention from lung tissue destruction by E64D in patients with cystic fibrosis

The predominant group of cysteine proteases contributing to the pathophysiology in CF are the cysteine cathepsins. The cysteine cathepsins are capable of degrading various extracellular matrix components, resulting in tissue destruction. Cathepsin S could be a player in early CF lung disease with extracellular cathepsin S concentrations correlating significantly with lung function decline and neutrophil recruitment into the airways [49] [50] [28] [29, 51-53].

The pharmacological inhibition of cathepsin S is described as associated with a reduction of neutrophil recruitment and lung tissue destruction. Cathepsin S may mediate inflammatory cell recruitment and mucin expression. In relation to airway ion transport, both cathepsin S and cathepsin B have been reported to activate ENaC, a sodium channel. Inhibition of this sodium channel by E64D could contribute to a less viscous airway secret. [50, 53]. As such, in concert with neutrophil elastase-mediated CFTR degradation, the inhibition of cysteine cathepsins by E64D may attenuate inflammation, tissue destruction, and the mucus dehydration.

### E64D prevents from developing of emphysema in patients with COPD

The loss of alveolar attachment sites is a morphological hallmark of emphysema in patients with chronic obstructive lung disease (COPD) [54]. Increased levels of cysteine proteases are associated with the development of emphysema. Mice given the cysteine protease inhibitor E64 have markedly attenuated emphysematous changes, implying that the inhibition of cysteine proteases by E64D is a target in the treatment of patients with emphysema [10].

### Treatment of asthma bronchiale using E64D

E64D could be effective in patients for a prevention and/or treatment of bronchial asthma, since our preclinical experiments showed an inhibition of IL-8 (a cytokine that is important for recruiting of neutrophilic granulocytes. In addition, in our experiments E64D inhibited Th17-differentiation and IL-17-release. The inhibition of both pathways may play a role in the treatment of bronchial asthma. In addition, E64D was found to polarize Th2-lymphocytes into Th1-lymphocytes. Hence, E64D may be used to treat both Th17- (neutrophilic-) as well as Th2-related asthma.

### Pharmaceutical composition comprising E64D and preferred application

The solution formulation of E64D is administered as a sterile, preservative-free, clear, colorless ethanol-based concentrated solution containing 50 µg/mL E64D in a single-use, clear glass vial with sufficient volume to withdraw 1.2 mL (= 60 µg E64D). In addition to the active ingredient, the solution formulation of E64D contains no other ingredients for inhalation. In the dosage regimen as tested, first, the above amount is inhaled in 12 healthy individuals (healthy volunteers) to exclude an adverse irritation of the airways. Lung function is assessed by body-plethysmography before inhalation and after inhalation of E64D

Then, for the treatment, the preferred dosage regimen is as follows: 3 times daily inhalation of 0.4 ml (15-to-30-minute period, total daily volume 1.2 ml) each time until the inhalation chamber is empty (the device has a sensor system that allows the device to shut down after complete inhalation). The expected E64D dosing duration is a total of 10 days. Inhaled doses are preferably administered by an M-neb^{®} dose device and over a 15-to-30-minute period three times a day. After inhalation, no residue remains in the nebulizer unit.

During treatment with E64D, the patient is admitted to an inpatient facility staffed and maintained by the requesting physician. A peripheral IV line or another venous catheter is maintained. Fluid resuscitation is available, if necessary, in the event of signs of renal failure or hypotension. Any fever is treated with acetaminophen (up to maximum permitted daily dose) and antibiotics as indicated.

Use of nonsteroidal anti-inflammatory medications and other nephrotoxic agents is avoided, if possible.

### Citations

1. Kasabova, M., et al., Regulation of TGF-β1-driven differentiation of human lung fibroblasts: emerging roles of cathepsin B and cystatin C. J Biol Chem, 2014. 289(23): p. 16239-51.
2. Lecaille, F., J. Kaleta, and D. Brömme, Human and parasitic papain-like cysteine proteases: their role in physiology and pathology and recent developments in inhibitor design. Chem Rev, 2002. 102(12): p. 4459-88.
3. Turk, V., et al., Cysteine cathepsins: from structure, function and regulation to new frontiers. Biochim Biophys Acta, 2012. 1824(1): p. 68-88.
4. Rawlings, N.D., A.J. Barrett, and A. Bateman, Using the MEROPS Database for Proteolytic Enzymes and Their Inhibitors and Substrates. Curr Protoc Bioinformatics, 2014. 48: p. 1.25.1-33.
5. Oelschlaegel, D., et al., Cathepsin Inhibition Modulates Metabolism and Polarization of Tumor-Associated Macrophages. Cancers, 2020. 12(9): p. 2579.
6. Repnik, U., et al., Cysteine Cathepsins Activate ELR Chemokines and Inactivate Non-ELR Chemokines. J Biol Chem, 2015. 290(22): p. 13800-11.
7. Li, X., et al., Cathepsin B Regulates Collagen Expression by Fibroblasts via Prolonging TLR2/NF-κB Activation. Oxid Med Cell Longev, 2016. 2016: p. 7894247.
8. Li, X., et al., Cysteinyl cathepsins: multifunctional enzymes in cardiovascular disease. Chonnam Med J, 2012. 48(2): p. 77-85.
9. Joyce, J.A., et al., Cathepsin cysteine proteases are effectors of invasive growth and angiogenesis during multistage tumorigenesis. Cancer Cell, 2004. 5(5): p. 443-53.
10. Wolters, P.J. and H.A. Chapman, Importance of lysosomal cysteine proteases in lung disease. Respir Res, 2000. 1(3): p. 170-7.
11. Dongre, A., et al., Cathepsin K in Lymphangioleiomyomatosis: LAM Cell-Fibroblast Interactions Enhance Protease Activity by Extracellular Acidification. Am J Pathol, 2017. 187(8): p. 1750-1762.
12. Ferreira-Gomes, M., et al., SARS-CoV-2 in severe COVID-19 induces a TGF-β-dominated chronic immune response that does not target itself. Nature Communications, 2021. 12(1): p. 1961.
13. Grant, R.A., et al., Circuits between infected macrophages and T cells in SARS-CoV-2 pneumonia. Nature, 2021. 590(7847): p. 635-641.
14. Xing, X., et al., IFN-γ+IL-17+Th17 cells regulate fibrosis through secreting IL-21 in systemic scleroderma. Journal of Cellular and Molecular Medicine, 2020. 24(23): p. 13600-13608.
15. Tramontana, M., et al., Nickel Allergy: Epidemiology, Pathomechanism, Clinical Patterns, Treatment, and Prevention Programs. Endocr Metab Immune Disord Drug Targets, 2020.
16. Li, F.Z., et al., Crosstalk between calpain activation and TGF-β1 augments collagen-I synthesis in pulmonary fibrosis. Biochim Biophys Acta, 2015. 1852(9): p. 1796-804.
17. Brown, R., et al., Cathepsin S: investigating an old player in lung disease pathogenesis, comorbidities, and potential therapeutics. Respir Res, 2020. 21(1): p. 111.
18. Kryczka, J. and J. Boncela, Proteases Revisited: Roles and Therapeutic Implications in Fibrosis. Mediators Inflamm, 2017. 2017: p. 2570154.
19. Twigg, M.S., et al., The Role of Serine Proteases and Antiproteases in the Cystic Fibrosis Lung. Mediators Inflamm, 2015. 2015: p. 293053.
20. Withana, N.P., et al., Non-invasive Imaging of Idiopathic Pulmonary Fibrosis Using Cathepsin Protease Probes. Sci Rep, 2016. 6: p. 19755.
21. Crouser, E.D., Role of imbalance between Th17 and regulatory T-cells in sarcoidosis. Curr Opin Pulm Med, 2018. 24(5): p. 521-526.
22. Bühling, F., et al., Cathepsin K--a marker of macrophage differentiation? J Pathol, 2001. 195(3): p. 375-82.
23. Murai, O., et al., Cathepsin B, D, and L regulation in cyclosporin A-mediated gingival hyperplasia of a patient with sarcoidosis. Clin Lab, 2011. 57(7-8)b: p. 535-41.
24. Morrone, C., et al., Cathepsin B promotes collagen biosynthesis driving Bronchiolitis Obliterans Syndrome. European Respiratory Journal, 2020: p. 2001416.
25. Rehm, S.R.T., et al., Premedication with a cathepsin C inhibitor alleviates early primary graft dysfunction in mouse recipients after lung transplantation. Sci Rep, 2019. 9(1): p. 9925.
26. Williams, A.S., et al., Role of cathepsin S in ozone-induced airway hyperresponsiveness and inflammation. Pulm Pharmacol Ther, 2009. 22(1): p. 27-32.
27. Hansel, N.N. and G.B. Diette, Gene expression profiling in human asthma. Proc Am Thorac Soc, 2007. 4(1): p. 32-6.
28. Kleyman, T.R. and D.C. Eaton, Regulating ENaC's gate. Am J Physiol Cell Physiol, 2020. 318(1): p. C150-c162.
29. Alli, A.A., et al., Cathepsin B is secreted apically from Xenopus 2F3 cells and cleaves the epithelial sodium channel (ENaC) to increase its activity. J Biol Chem, 2012. 287(36): p. 30073-83.
30. Small, D.M., et al., Targeting of cathepsin S reduces cystic fibrosis-like lung disease. European Respiratory Journal, 2019. 53(3): p. 1801523.
31. Tse, G.M., et al., Pulmonary pathological features in coronavirus associated severe acute respiratory syndrome (SARS). J Clin Pathol, 2004. 57(3): p. 260-5.
32. Zenewicz, L.A., IL-22: There Is a Gap in Our Knowledge. ImmunoHorizons, 2018. 2(6): p. 198-207.
33. Huang, C., et al., Clinical features of patients infected with 2019 novel coronavirus in Wuhan, China. Lancet, 2020. 395(10223): p. 497-506.
34. Xu, Z., et al., Pathological findings of COVID-19 associated with acute respiratory distress syndrome. Lancet Respir Med, 2020. 8(4): p. 420-422.
35. Xu, N., et al., Increased levels of lysosomal cysteinyl cathepsins in human varicose veins: a histology study. Thromb Haemost, 2014. 111(2): p. 333-44.
36. Joean, O., et al., Suppression of Th17-polarized airway inflammation by rapamycin. Sci Rep, 2017. 7(1): p. 15336.
37. Hou, L., et al., The protease cathepsin L regulates Th17 cell differentiation. J Autoimmun, 2015. 65: p. 56-63.
38. Bermejo-Martin, J.F., et al., Th1 and Th17 hypercytokinemia as early host response signature in severe pandemic influenza. Critical Care, 2009. 13(6): p. R201.
39. Hamon, Y., et al., Neutrophilic Cathepsin C Is Maturated by a Multistep Proteolytic Process and Secreted by Activated Cells during Inflammatory Lung Diseases. J Biol Chem, 2016. 291(16): p. 8486-99.
40. Korkmaz, B., et al., Neutrophil elastase, proteinase 3, and cathepsin G as therapeutic targets in human diseases. Pharmacol Rev, 2010. 62(4): p. 726-59.
41. Korkmaz, B., et al., Lung Protection by Cathepsin C Inhibition: A New Hope for COVID-19 and ARDS? J Med Chem, 2020. 63(22): p. 13258-13265.
42. Tsubokawa, T., et al., Cathepsin and calpain inhibitor E64d attenuates matrix metalloproteinase-9 activity after focal cerebral ischemia in rats. Stroke, 2006. 37(7): p. 1888-94.
43. Hoffmann, M., et al., SARS-CoV-2 Cell Entry Depends on ACE2 and TMPRSS2 and Is Blocked by a Clinically Proven Protease Inhibitor. Cell, 2020. 181(2): p. 271-280.e8.
44. Bühling, F., et al., Pivotal role of cathepsin K in lung fibrosis. Am J Pathol, 2004. 164(6): p. 2203-16.
45. She, Y.X., Q.Y. Yu, and X.X. Tang, Role of interleukins in the pathogenesis of pulmonary fibrosis. Cell Death Discov, 2021. 7(1): p. 52.
46. Li, C., et al., IL-17 response mediates acute lung injury induced by the 2009 Pandemic Influenza A (H1N1) Virus. Cell Research, 2012. 22(3): p. 528-538.
47. Wu, D. and X.O. Yang, TH17 responses in cytokine storm of COVID-19: An emerging target of JAK2 inhibitor Fedratinib. J Microbiol Immunol Infect, 2020. 53(3): p. 368-370.
48. Swanson, K.V., M. Deng, and J.P.Y. Ting, The NLRP3 inflammasome: molecular activation and regulation to therapeutics. Nature Reviews Immunology, 2019. 19(8): p. 477-489.
49. Weldon, S., et al., miR-31 dysregulation in cystic fibrosis airways contributes to increased pulmonary cathepsin S production. Am J Respir Crit Care Med, 2014. 190(2): p. 165-74.
50. Haerteis, S., et al., Proteolytic activation of the epithelial sodium channel (ENaC) by the cysteine protease cathepsin-S. Pflugers Arch, 2012. 464(4): p. 353-65.
51. Mall, M.A., ENaC inhibition in cystic fibrosis: potential role in the new era of CFTR modulator therapies. Eur Respir J, 2020. 56(6).
52. McKelvey, M.C., et al., Targeting Proteases in Cystic Fibrosis Lung Disease. Paradigms, Progress, and Potential. Am J Respir Crit Care Med, 2020. 201(2): p. 141-147.
53. Tan, C.D., et al., Cathepsin B contributes to Na+ hyperabsorption in cystic fibrosis airway epithelial cultures. J Physiol, 2014. 592(23): p. 5251-68.
54. Zheng, T., et al., Inducible targeting of IL-13 to the adult lung causes matrix metalloproteinase- and cathepsin-dependent emphysema. J Clin Invest, 2000. 106(9): p. 1081-93.

## Claims

1. A compound according to Formula (I), 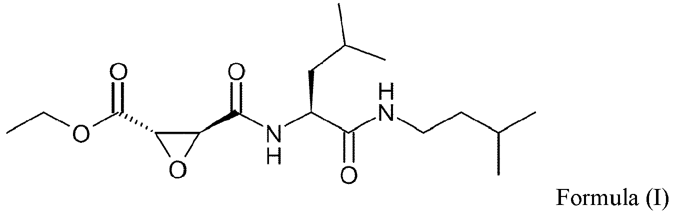 a physiologically acceptable salt, a solvate, or a hydrate thereof for use in the prevention and/or treatment of an inflammatory and/or other fibrotic lung disease in a mammalian subject, such as a human, wherein said compound, the physiologically acceptable salt, the solvate, or the hydrate thereof is administered by inhalation as a pharmaceutical composition comprising a suitable solvent as a carrier.

2. The compound for use according to claim 1, wherein said inflammatory and/or other fibrotic lung disease is an interstitial and/or neoplastic autoimmune lung disease, and/or said prevention and/or treatment is against exacerbations or severe inflammation in said disorder, and/or to inhibit the IL-17 induced scarring of the lung tissue, and preferably to avoid irreversible airway remodeling up to terminal fibrosis of the lung.

3. The compound for use according to claim 1 or 2, wherein said disease or condition is selected from the group consisting of lung fibrosis, such as, for example, IPF, NSIP; AIP, LIP, RB-ILD, histiocytosis, fibrotic autoimmune disorders, systemic sclerosis, rheumatoid arthritis with fibrotic lung involvement, asthma bronchiale, in particular Th1- and/or Th2- and/or Th17-related asthma bronchiale, Sjogren's syndrome, ANA and ANCA associated vasculitis, lupus erythematosus with fibrotic lung involvement, hypersensitivity pneumonitis, Granulomatosis with polyangiitis, eosinophilic granulomatosis and polyangiitis, sarcoidosis, beryllium disease, cystic fibrosis, check point inhibitor induced pneumonitis, radiation induced pneumonitis, graft versus host disease, bronchiolitis obliterans syndrome, chronic lung allograft dysfunction, IgG4-associated diseases of the lungs, lymphangioleiomyomatosis, amyloidosis of the lungs, lung metastases derived from lung cancer, breast cancer, colon cancer or renal cell cancer, COPD, emphysema, and ARDS in patients with severe viral and bacterial pneumonia.

4. The compound for use according to any one of claims 1 to 3, wherein said prevention and/or treatment is in combination with another therapy, for example selected from at least one of interferon alpha 2a or 2b, inclusive any pegylated versions, serine protease inhibitors, cysteine protease inhibitors, and/or type II transmembrane protease (TMPRSS2) inhibitors, in particular camostat, inhalable corticosteroids, vasoactive intestinal peptide (VIP) and furin inhibitors.

5. The compound for use according to claim 4, wherein said combination compound is provided as a suitable pharmaceutical composition, such as a tablet, capsule, injection, granule, powder, sachet, reconstitutable powder, dry powder inhaler, inhalation, and/or chewable.

6. The compound for use according to anyone claims 1 to 5, wherein said suitable solvent is an alcohol, such as ethanol.

7. The compound for use according to anyone claims 1 to 6, wherein said inhalation is through a breath-triggered nebulizer.

8. A method for preventing and/or treating of an inflammatory and/or other fibrotic lung disease in a mammalian subject, such as a human, comprising administering to said mammal an effective amount of a compound according to Formula (I), 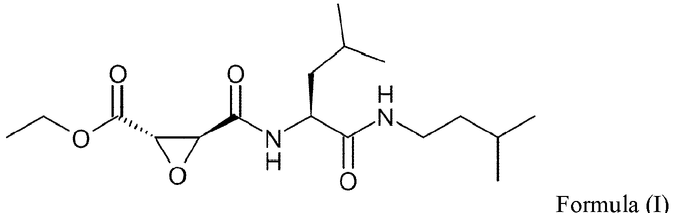 a physiologically acceptable salt, a solvate, or a hydrate thereof, wherein said compound is administered by inhalation as a pharmaceutical composition comprising a suitable solvent as a carrier, wherein preferably said suitable solvent is an alcohol, such as ethanol.

9. The method according to claim 8, wherein said inflammatory and/or other fibrotic lung disease is an interstitial and/or neoplastic autoimmune lung disease, and/or said prevention and/or treatment is against exacerbations or severe inflammation in said disorder, and/or to inhibit the IL-17 induced scarring of the lung tissue, and preferably in order to avoid irreversible airway remodeling up to terminal fibrosis of the lung.

10. The method according to claim 8 or 9, wherein said disease or condition is selected from the group consisting of lung fibrosis, such as, for example, IPF, NSIP; AIP, LIP, RB-ILD, histiocytosis, fibrotic autoimmune disorders, systemic sclerosis, rheumatoid arthritis with fibrotic lung involvement, asthma bronchiale, in particular Th1- and/or Th2-, Th17- and/or related asthma bronchiale, Sjogren's syndrome, ANA and ANCA associated vasculitis, lupus erythematosus with fibrotic lung involvement, hypersensitivity pneumonitis, Granulomatosis with polyangiitis, eosinophilic granulomatosis and polyangiitis, sarcoidosis, beryllium disease, cystic fibrosis, check point inhibitor induced pneumonitis, radiation induced pneumonitis, graft versus host disease, bronchiolitis obliterans syndrome, chronic lung allograft dysfunction, IgG4-associated diseases of the lungs, lymphangioleiomyomatosis, amyloidosis of the lungs, lung metastases derived from lung cancer, breast cancer, colon cancer or renal cell cancer, COPD, emphysema, and ARDS in patients with severe viral and bacterial pneumonia.

11. The method according to any one of claims 8 to 10, wherein said prevention and/or treatment is in combination with another therapy, for example selected from at least one of interferon alpha 2a or 2b, inclusive any pegylated versions, serine protease inhibitors, cysteine protease inhibitors, and/or type II transmembrane protease (TMPRSS2) inhibitors, in particular camostat, inhalable corticosteroids, vasointestinal peptide (VIP) and furin inhibitors.

12. The method according to any one of claims 8 to 11, wherein said method further comprises a monitoring step comprising an analysis selected from serum chemistries including electrolytes, renal function tests, such as creatinine, CrCL, and BUN, liver function tests, such as ALT, AST, total bilirubin, and alkaline phosphatase, hematology, such as complete blood count and prothrombin time, and urinalysis in a sample obtained from said mammalian subject, and comparing said analysis to the analysis of an earlier sample from said mammal and/or a control sample.
